Europäisches Patentamt

European Patent Office

Office européen des brevets

(19)

Veröffentlichungsnummer: **0 268 193**

**A2**

## EUROPÄISCHE PATENTANMELDUNG

Anmeldenummer: 87116625.2

Anmeldetag: 11.11.87

Int. Cl.4 **C12N 9/24** , C12N 9 28 . C12N 9 34 . C12N 9 44

Der Mikroorganismus ist bei der Deutsche Sammlung von Microorganismen unter der Nummer DSM 3896 hinterlegt worden.

Priorität: 17.11.86 DE 3639267

Veröffentlichungstag der Anmeldung:
25.05.88 Patentblatt 88/21

Benannte Vertragsstaaten:
AT BE CH DE ES FR GB IT LI NL

Anmelder: Kali-Chemie Aktiengesellschaft
Postfach 220 Hans-Böckler-Allee 20
D-3000 Hannover 1(DE)

Erfinder: Antranikian, Garabed, Dr.
Grisebachstrasse 8
D-3400 Göttingen(DE)
Erfinder: Gottschalk, Gerhard, Prof. Dr.
Grisebachstrasse 8
D-3400 Göttingen(DE)

Vertreter: Lauer, Dieter. Dr.
c/o Kali-Chemie AG Postfach 220
Hans-Böckler-Allee 20
D-3000 Hannover 1(DE)

Verfahren zur Förderung der Exkretion von amylolytischen Enzymen aus Bakterien sowie nach dem Verfahren erhaltene Enzyme.

Die Erfindung betrifft ein Verfahren zur Förderung der Exkretion von amylolytischen Enzymen aus thermophilen, stärkeabbauenden, die genannten Enzyme bildenden anaeroben Bakterien sowie die Enzyme, die nach dem Verfahren erhältlich sind. nämlich Pullulanasen, alpha-Amylase und Glucoamylase.

EP 0 268 193 A2

## Verfahren zur Förderung der Exkretion von amylolytischen Enzymen aus Bakterien sowie nach dem Verfahren erhaltene Enzyme

Die Erfindung betrifft ein Verfahren zur Förderung der Exkretion von amylolytischen Enzymen (Amylasen und Pullulanasen) aus thermophilen, die genannten Enzyme bildenden anaeroben Bakterien sowie zwei Enzyme, die nach dem Verfahren erhältlich sind, nämlich Pullulanase und alpha-Amylase.

Die Bildung von amylolytischen, d.h. Stärke abbauenden Enzymen ist bekannt. So wird nach WO-A-86.01 831 Clostridium thermohydrosulfuricum ATCC 33 223 ansatzweise auf einem Kulturmedium mit einem Gehalt an 0,5 % löslicher Stärke in Batchkultur kultiviert, wobei zwar Amylase, Pullulanase und Glucoamylase gebildet, jedoch nicht in das Kulturmedium ausgeschieden werden; vgl. auch Appl. Envir. Microbiol., 49 (1985) 1168-1173. Nach WO-A-86.01 832 wird Clostridium thermosulfurogenes ATCC 33 743 = DSM 2 229 ansatzweise in einem Kulturmedium mit 0,5 % löslicher Stärke kultiviert, wobei Amylase, Pullulanase und Glucoamylase als amylolytische Enzyme gebildet werden, jedoch nur das zuerst genannte Enzym in das Kulturmedium austritt.

Aufgabe der Erfindung ist es, ein Verfahren vorzusehen, bei dem amylolytische Enzyme bei der Kultivierung von thermophilen, die genannten Enzyme bildenden anaeroben Bakterien in hohem Maße in das Kulturmedium ausgeschieden werden. Ein derartiges Verfahren bietet den Vorteil, daß die Zellen nicht zertrümmert und die Enzyme nicht von den Zelltrümmern und störenden Komponenten der Zellflüssigkeit abgetrennt werden müssen.

Dazu wird erfindungsgemäß ein Verfahren zur Förderung der Exkretion von amylolytischen Enzymen aus thermophilen, die gennanten Enzyme bildenden anaeroben Bakterien in das Kohlenstoff-Quellen, Stickstoff-Quellen, Mineralsalze und gegebenenfalls Vitamine enthaltende Kulturmedium vorgeschlagen, das dadurch gekennzeichnet ist, daß man
-das Verfahren kontinuierlich durchführt,
-als Kohlenstoff-Quelle ein höheres Saccharid verwendet und
-unter in Bezug auf dieses höhere Saccharid limitierenden Bedingungen kultiviert.

Der Fachmann ist mit der Isolierung und Kultivierung von thermophilen, amylolytische Enzyme bildenden anaeroben Bakterien vertraut. Brauchbare Stämme finden sich in der Gattung Clostridium, beispielsweise ihrer Art Cl. thermosulfurogenes, wie ATCC 33743 = DSM 2229, ihrer Art Cl. thermohydrosulfuricum, wie ATCC 33223, DSM 567, DSM 2247 und DSM 2355, ihrer Art Cl. thermosaccharolyticum, wie DSM 571 und DSM 572, und ihrer Art Cl. spec. DSM 3896, in der Gattung Thermoanaerobacter, beispielsweise ihrer Art Th. ethanolicus, wie DSM 2246, in der Gattung Thermobacteroides, beispielsweise ihrer Art Th. acetoethylicus, wie DSM 2359, und in der Gat tung Thermoanaerobicum, beispielsweise ihrer Art Th. brockii, wie DSM 1457.

Der Ausdruck "höheres Saccharid" ist im vorliegenden Zusammenhang so zu verstehen, daß Monosaccharide ausgenommen sind. Beispiele für höhere Saccharide sind Polysaccharide, wie Stärke, Dextrin, Dextran oder Pullulan, und Oligosaccharide, wie Trisaccharide, z.B. Maltotriose, oder Disaccharide, z.B. Maltose, und deren Gemische.

Es ist dem Fachmann zuzumuten, die geeignete Konzentration des höheren Saccharids, den geeigneten pH-Wert und die geeignete Durchflußrate (Verdünnungsrate) zu ermitteln.

Lediglich zur Veranschaulichung sei angeführt, daß das Verfahren bei einer Konzentration an höherem Saccharid von 0,1 bis 3, vorzusgweise 0,2 bis 1,5 und insbesondere 0,5 bis 1,2 % (Gewicht/Volumen) durchgeführt werden kann.

Zur Veranschaulichung sei ferner ein pH-Wert von beispielsweise 4 bis 8, vorzugsweise 5,0 bis 7,5 und insbesondere 5,5 bis 7,0 genannt.

Zur Veranschaulichung sei schließlich eine Durchflußrate von beispielsweise 0,01 bis 0,4, insbesondere 0,02 bis 0,3 vorzugsweise 0,02 bis 0,2 und besonders bevorzugt von 0,03 bis 0,15 $h^{-1}$ genannt.

Hinsichtlich der Wahl einer geeigneten Temperatur wird sich der Fachmann danach richten, daß das Temperatur-Minimum durch zu langsames Zellwachstum und das Temperatur-Maximum durch Proteindenaturierung und Absterben der Zellen vorgegeben werden.

Gegenstand der Erfindung sind ferner eine Pullulanase und eine alpha-Amylase mit den Kennzeichen der Ansprüche 6 und 7. Da die beiden Enzyme ihr Aktivitätsoptimum jeweils im Bereich von 5,0 bis 5,5 bei Temperaturen von 70 °C (Pullulanase) bzw. 60 °C (alpha-Amylase) zeigen, lassen sie sich vorteilhaft technisch in ein und demselben Reaktionsmedium verwenden.

Nachstehend wird die Erfindung durch Beispiele anhand von Figuren näher erläutert.

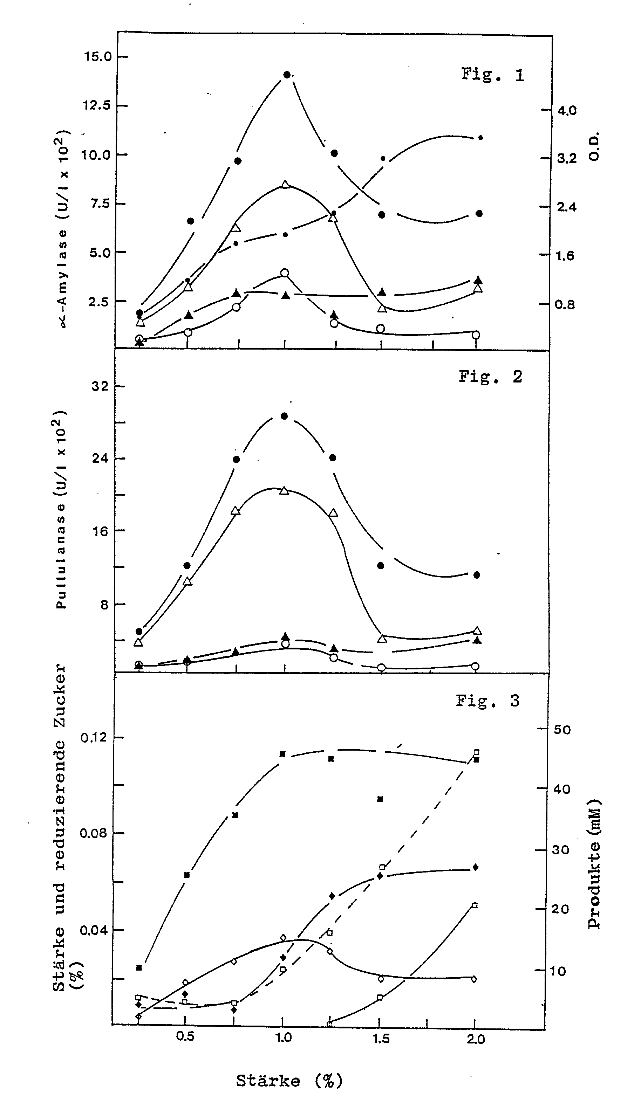

Fig. 1-3. Der Einfluß der Stärkekonzentration auf alpha-Amylase-und Pullulanase-Bildung und Exkretion in kontinuierlicher Kultur. Die Experimente wurden mit Clostridium spec. DSM 3896 bei einem pH-Wert von 5,9 und einer Temperatur von 60 °C durchgeführt. Die Durchflußrate betrug 0,075

$h^{-1}$.

△ extrazelluläre Enzymkonzentration (Einheiten/l)
▲ zellgebundene Enzymkonzentration (E/l)
○ intrazelluläre Enzymkonzentration (E/l)
● gesamte Enzymkonzentration (E/l)
● optische Dichte bei 580 nm
□-□ restliche Stärke (%)
□.□ reduzierende Zucker (%)
◊ Acetate (mM)
■ Ethanol (mM)
♦ Laktat (mM)

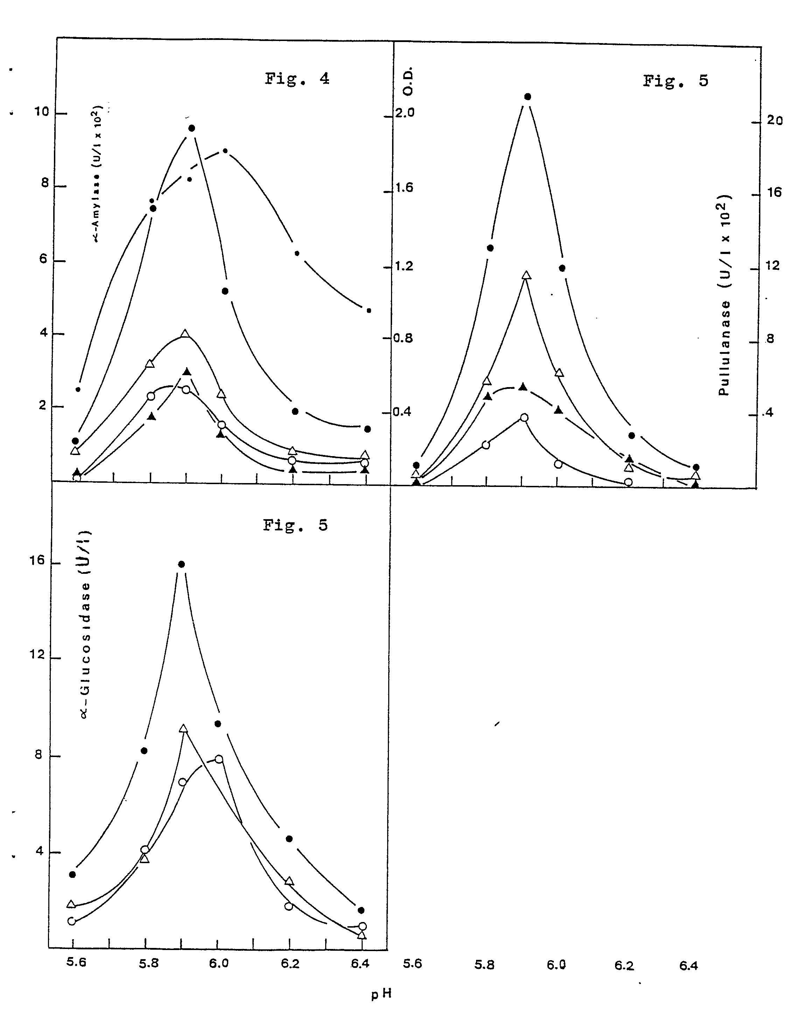

Fig. 4-6. Der Einfluß des pH-Wertes auf alpha-Amylase-, Pullulanase-und alpha-Glucosidase-Bildung und Exkretion in kontinuierlicher Kultur. Die Experimente wurden mit Clostridium spec. DSM 3896 in Gegenwart von 1 % Stärke bei 60 °C durchgeführt. Die Durchflußrate betrug 0.075 $h^{-1}$. Symbole wie in Fig. 1-3.

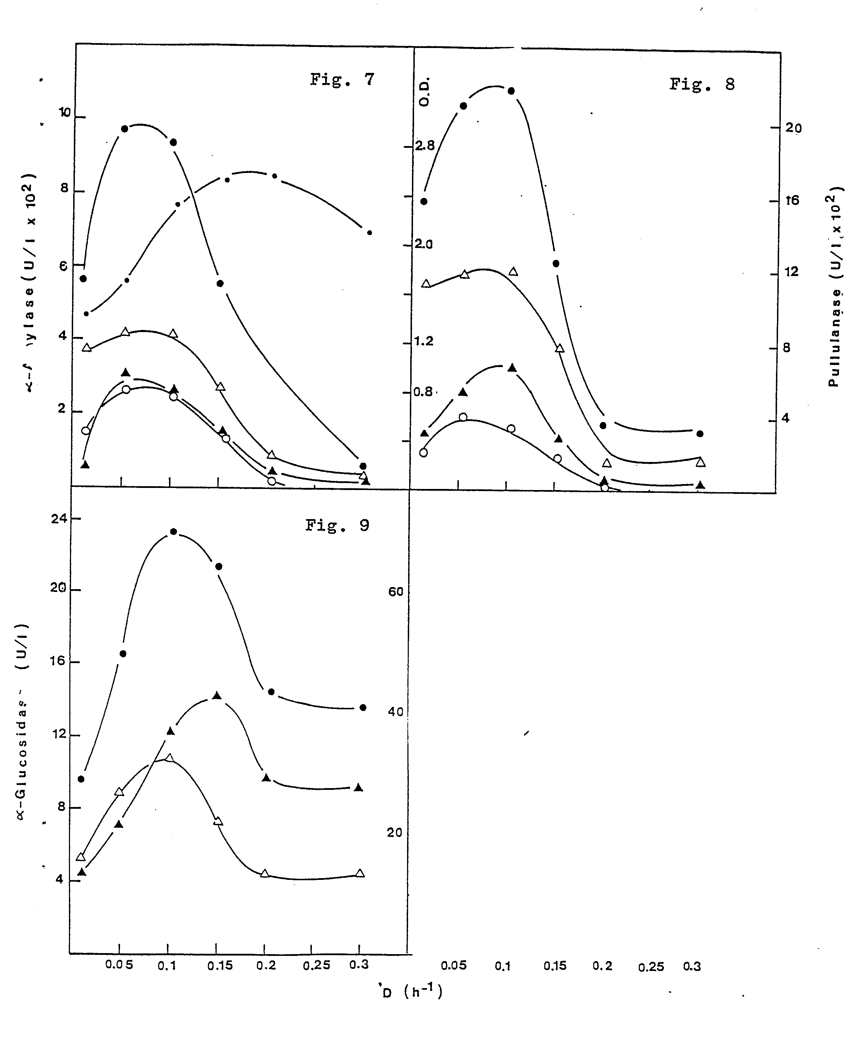

Fig. 7-9. Der Einfluß der Durchflußrate auf alpha-Amylase-, Pullulanase-und alpha-Glucosidase-Bildung und Exkretion in kontinuierlicher Kultur. Die Experimente wurden mit Clo-stridium spec. DSM 3896 in Gegenwart von 1 % Stärke bei einem pH-Wert von 5,9 und einer Temperatur von 60 °C durchgeführt. Symbole wie in Fig. 1-3.

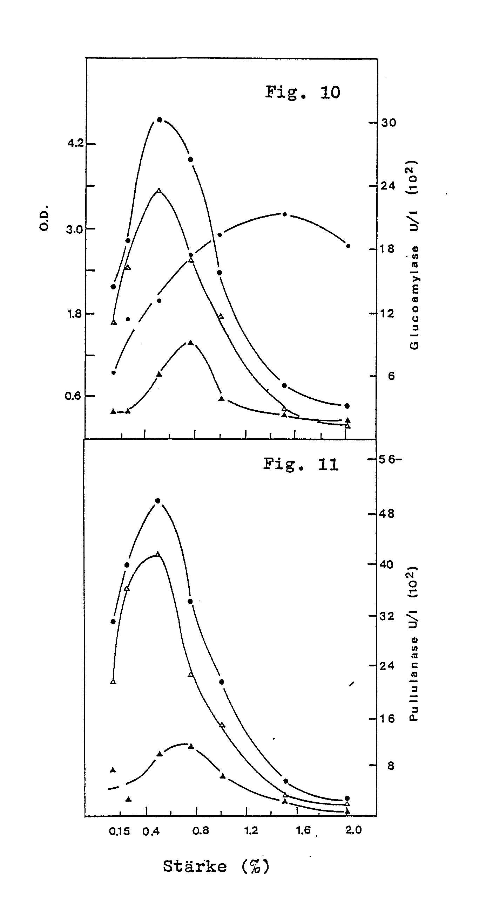

Fig. 10-11. Der Einfluß der Stärkekonzentration auf Glucoamylase-und Pullulanase-Bildung und Exkretion in kontinuierlicher Kultur. Die Experimente wurden mit Clostridium thermohydrosulfuricum DSM 567 bei einem pH-Wert von 6,5 und einer Temperatur von 60 °C durchgeführt. Die Durchflußrate betrug 0,05 $h^{-1}$. Symbole wie in Fig. 1-3.

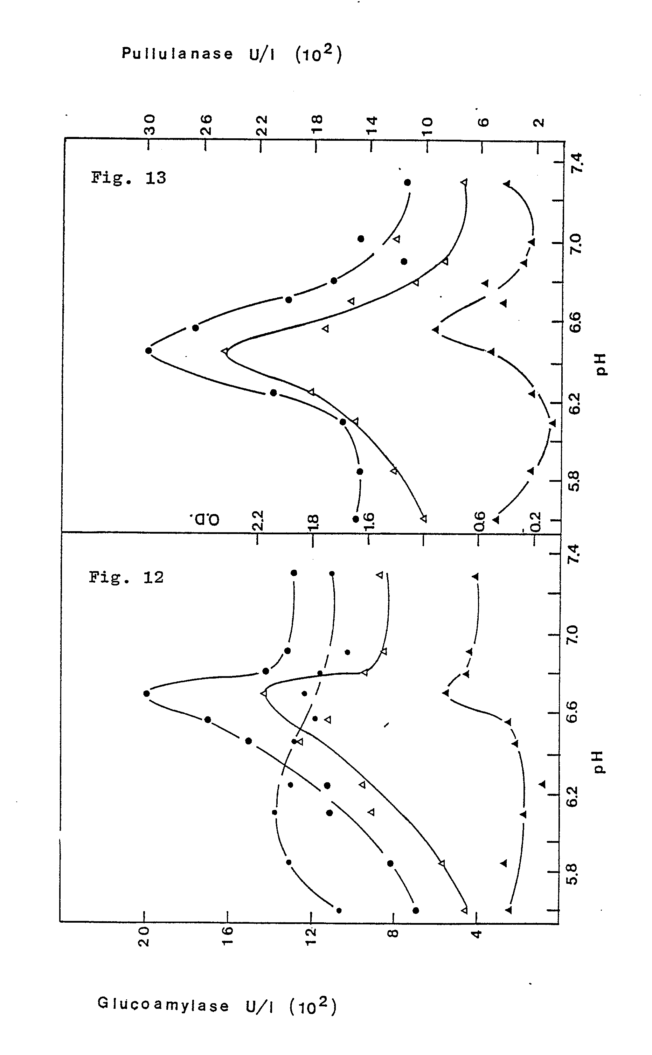

Fig. 12-13. Der Einfluß des pH-Wertes auf Glucoamylase-und Pullulanase-Bildung und Exkretion in kontinuierlicher Kultur. Die Experimente wurden mit Clostridium thermohydrosulfuricum DSM 567 in Gegenwart von 0,5 % Stärke bei 60 °C durchgeführt. Die Durchflußrate betrug 0,05 $h^{-1}$. Symbole wie in Fig. 1-3.

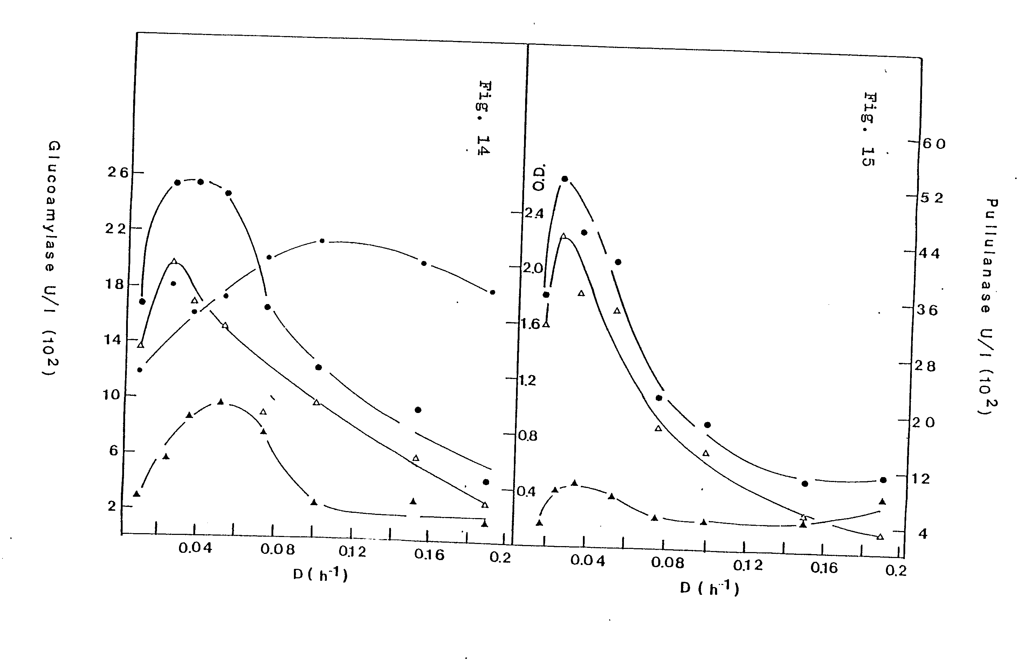

Fig. 14-15. Der Einfluß der Durchflußrate auf Glucoamylase-und Pullulanase-Bildung und Exkretion in kontinuierlicher Kultur. Die Experimente wurden mit Clostridium thermohydrosulfuricum DSM 567 in Gegenwart von 0,5 % Stärke bei einem pH-Wert von 6,5 und einer Temperatur von 60 °C durchgeführt. Symbole wie in Fig. 1-3.

Beispiel 1 (Figuren 1 bis 9)

Das kontinuierliche Verfahren wurde mit Clostridium spec. DSM 3896 (hinterlegt am 10.11.1986 bei der Deutsche Sammlung von Mikroorganismen) (Em 1) mit einem definierten Medium durchgeführt, das die folgenden Bestandteile enthielt (% Gewicht/Volumen): $KH_2PO_4$ 0,68; $(NH_4)_2SO_4$ 0,1;

$MgCl_2.6H_2O$ 0,016; $CoCl_2.6H_2O$ 0,0012; und Cystein.HCl 0,05. Pro l Medium wurden jeweils 1 ml einer Vitamin-Lösung und einer Spurenelement-Lösung zugegeben. Der pH betrug 6,5. Das definierte Medium wurde mit den in Tabelle 1 angegebenen Sacchariden ergänzt.

Die Versuche wurden in 2-1-oder 5-1-Fermentoren bei einem Kulturvolumen von 1 l bis 2 l bei 60 °C durchgeführt. Die Kulturen wurden durch einen kontinuierlichen Stickstoffstrom anaerob gehalten. Der Stickstoff wurde sterilisiert, indem man ihn durch ein sterilisiertes Baumwoll-Filter strömen ließ. Die Kulturen wurden mit 150 U/min gerührt. Der ph-Wert wurde mit einer Glaselektode gemessen und bei dem vorgegebenen Wert gehalten, indem man automatisch 2 N KOH zugab. Nachdem man die stabile Phase erreicht hatte (bei mindestens 6 Volumenwechseln), wurden drei 10-ml-Proben in Abständen von 24 h genommen und die Aktivitäten der amylolytischen Enzyme sowie ihre Lokalisierung untersucht. Ferner wurden die Fermentationsprodukte, die Substratkonzentrationen, der pH-Wert und die optische Dichte bei 580 nm gemessen.

Der Einfluß der Saccharid-Konzentration, des pH-Wertes und der Durchflußrate auf die Ausscheidung amylolytischer Enzyme in das Kulturmedium sind den Figuren 1 bis 9 am Beispiel von Stärke als Substrat zu entnehmen.

Tabelle 1 kann man den Einfluß verschiedener Saccharide auf die Ausscheidung amylolytischer Enzyme entnehmen.

Beispiel 2 (Figuren 10 bis 15)

Beispiel 1 wurde mit Clostridium thermohydrosulfurium DSM 567 mit Stärke als Substrat wiederholt. Das Medium enthielt folgende Bestandteile (% Gewicht/Volumen): 0,25 Trypton; 0,1 Hefe-Extrakt; 0,33 $KH_2PO_4$; 0,016 $MgCl_2$; 0,0012 $CoCl_2.6H_2O$; 0,05 Cystein.HCl. Pro l Medium wurde jeweils 1 ml einer Vitaminlösung und einer Spurenelementlösung zugegeben.

Der Einfluß der Saccharid-Konzentration, des pH-Wertes und der Durchflußrate auf die Ausscheidung amylolytischer Enzyme (Glucoamylase und Pullulanase) in das Kulturmedium sind den Figuren 10 bis 15 zu entnehmen.

Beispiel 3

Beispiel 2 wurde für folgende Mikroorganismen mit Stärke als Substrat wiederholt:
Clostridium thermoysulfurogenes DSM 2229
Clostridium thermohydrosulfuricum DSM 2247
Clostridium thermohydrosulfuricum DSM 2355

Clostridium thermosaccharolyticum DSM 571
Clostridium thermosaccharolyticum DSM 572
Thermoanaerobacter ethanolicus DSM 2246
Thermobacteroides acetoethylicus DSM 2359
Thermoanaerobium brockii DSM 1457

Bei den Versuchen mit den genannten Stämmen wurde bestätigt, daß sich die Exkretion von amylolytischen Enzymen durch Limitierung des höheren Saccharids als Kohlenstoff-Quelle bei den im Beispiel 1 angegebenen Werten für den pH und die Durchflußrate fördern läßt.

Tabelle 1: Einfluß verschiedener Saccharide auf Enzym-Bildung und -Exkretion bei Clostridium spec. DSM 3896 unter Substratlimitierung

| Substrate | Substrat-Konzentration (% Gewicht/Vol.) | Gesamtaktivität (E/l) | | Extrazellulares Enzym | | | | Produktivität $(E.l^{-1}.h^{-1})$ | |
|---|---|---|---|---|---|---|---|---|---|
| | | Amylase | Pullulanase | Amylase | | Pullulanase | | Amylase | Pullulanase |
| | | | | (E/l) | (%) | (E/l) | (%) | | |
| Stärke | 1 | 1452 | 2867 | 852 | 55 | 2058 | 70 | 109 | 215 |
| Stärke | 2 | 740 | 1150 | 350 | 48 | 610 | 53 | 55 | 85 |
| Stärke + Hefeextrakt | 1+ 0.2 | 1007 | 2237 | 284 | 28 | 1131 | 50 | 75 | 168 |
| Dextrin | 1 | 1295 | 3100 | 685 | 53 | 1692 | 55 | 97 | 232 |
| Maltose | 1 | 730 | 1924 | 310 | 42 | 634 | 33 | 55 | 144 |
| Glucose | 1 | 140 | 215 | 106 | 75 | 160 | 75 | 10 | 16 |

0 268 193

**Ansprüche**

1. Verfahren zur Förderung der Exkretion von amylolytischen Enzymen aus thermophilen, die genannten Enzyme bildenden anaeroben Bakterien in das Kohlenstoff-Quellen, Stickstoff-Qeullen, Mineralsalze und gegebenenfalls Vitamine enthaltende Kulturmedium, dadurch gekennzeichnet, daß man
--das Verfahren kontinuierlich durchführt und
--als Kohlenstoff-Quelle ein höheres Saccharid verwendet und
--unter in Bezug auf das höhere Saccharid limitierenden Bedingungen kultiviert.

2. Verfahren nach Anspruch 1, dadurch gekennzeichnet, daß man als höheres Saccharid ein Polysaccharid, wie Stärke, Dextrin, Dextran oder Pullulan, oder ein Oligosaccharid, z.B. ein Trisaccharid, beispielsweise Maltotriose, oder z.B. ein Disaccharid, beispielsweise Maltose, oder deren Gemische verwendet.

3. Verfahren nach Anspruch 1 oder 2, dadurch gekennzeichnet, daß man das Verfahren bei einer Konzentration an höherem Saccharid von 0.1 bis 3, vorzugsweise 0,2 bis 1,5 und insbesondere 0,5 bis 1,2 % (Gewicht/Volumen) durchführt.

4. Verfahren nach einem der vorhergehenden Ansprüche, dadurch gekennzeichnet, daß man das Verfahren bei einem pH von 4 bis 8, vorzugsweise 5,0 bis 7,5 und insbesondere 5,5 bis 7,0 durchführt.

5. Verfahren nach einem der vorhergehenden Ansprüche, dadurch gekennzeichnet, daß man das Verfahren bei einer Durchflußrate von 0,01 bis 0,4, insbesondere 0,02 bis 0,3, vorzugsweise 0,02 bis 0,2 und besonders bevorzugt 0,03 bis 0,15 $h^{-1}$ durchführt.

6. Pullulanase, erhältlich durch Isolierung aus einem amylolytische Enzyme enthaltenden Kulturmedium, das bei dem Verfahren gemäß einem der Ansprüche 1 bis 5 bei Verwendung von Clostridium spec. DSM 3896 als Bakterium anfällt, und mit den folgenden Merkmalen:
Aktivitätsoptimum bei 70 °C und pH 5,0 bis 5,5
kein Aktivitätsverlust nach 1 Tag bei 60 °C unter aeroben Bedingungen ohne Substratzugabe.

7. alpha-Amylase, erhältlich durch Isolierung aus einem amylolytische Enzyme enthaltenden Kulturmedium, das bei dem Verfahren gemäß einem der Ansprüche 1 bis 5 bei Verwendung von Clostridium spec. DSM 3896 als Bakterium anfällt, und mit dem folgenden Merkmal:
Aktivitätsoptimum bei 60 °C und pH 5,0 bis 5,5.

Fig. 1

Fig. 2

Fig. 3

Fig. 4

Fig. 5

Fig. 5

Fig. 7

Fig. 8

Fig. 9

0 268 193

Fig. 10

Fig. 11

Stärke (%)

Pullulanase U/l (10$^2$)

Fig. 13

Fig. 12

O.D.

pH

pH

Glucoamylase U/l (10$^2$)

Fig. 14

Fig. 15